# FASCICULE DE BREVET EUROPEEN

(11) **EP 1 145 710 B1**
(45) Date de publication et mention de la délivrance du brevet: **02.11.2006**
(21) Numéro de dépôt: 01400781.9
(22) Date de dépôt: 27.03.2001
(51) Int. Cl.: A61K 8/67, A61Q 19/00

(54) **Utilisation de dérivés d'acide ascorbique pour augmenter la synthèse des céramides épidermiques**
Verwendung von Ascorbinsäurederivaten, um die Synthese von epidermalen Ceramiden zu erhöhen
Use of ascorbic acid derivatives for increasing epidermal ceramides synthesis

(30) Priorité: 10.04.2000 FR 0004574
(43) Date de publication de la demande: 17.10.2001
(73) Titulaire: L'ORÉAL, 75008 Paris (FR)
(72) Inventeur: Castiel, Isabelle, 78350 Jouy-en-Josas (FR); Ferraris, Corinne, 75017 Paris (FR)
(74) Mandataire: Kromer, Christophe

(56) Documents cités:
- EP-A- 0 487 404
- EP-A- 0 875 514
- EP-A- 0 916 662
- EP-A- 0 917 871
- EP-A- 1 077 066
- WO-A-99/26588
- US-A- 5 536 500
- US-A- 5 730 972
- M. PONEC ET AL.: "The formation of competent barrier lipids in reconstructed human epidermis requires the presence of vitamin C" THE JOURNAL OF INVESTIGATIVE DERMATOLOGY, vol. 109, no. 3, 1997, pages 348-355, XP000972591
- P.MORGANTI ET AL.: "Safety evaluation of phytosphingosine and ceramides of pharmaceutical grade" JOURNAL OG APPLIED COSMETOLOGY, vol. 17, no. 1, 1999, pages 1-9, XP000972602
- DATABASE CHEMICAL ABSTRACTS [en ligne] STN; access number 124: 56 571, XP002171906 & JP 07 206840 A (KANTO DENKA KKK) 8 août 1995 (1995-08-08)
- DATABASE CHEMICAL ABSTRACTS [en ligne] STN; access number 105: 196 988, XP002171907 & JP 61 085308 A (KANEBO, LTD) 30 avril 1986 (1986-04-30)
- * The Merck Manual , 17th ed., M. Beers et al. ed., 1999, pp788-791, 816-821.

## Description

L'invention se rapporte à l'utilisation de certains dérivés d'acide ascorbique pour la fabrication d'une composition destinée à augmenter la synthèse des céramides épidermiques.

La peau humaine est constituée de deux compartiments à savoir un compartiment profond, le derme, et un compartiment superficiel, l'épiderme.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale, composants synthétisés par le fibroblaste. On y trouve aussi des leucocytes, des mastocytes
ou encore des macrophages tissulaires. Il contient également des vaisseaux sanguins et des fibres nerveuses.

L'épiderme est en contact avec l'environnement extérieur. Son rôle consiste à protéger l'organisme de la déshydratation et des agressions extérieures, qu'elles soient chimiques, mécaniques, physiques ou infectieuses.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Les cellules constituant l'épiderme sont délimitées par un domaine lipidique. Au cours de la différenciation, les phospholipides dont le rôle consiste à élaborer la structure fluide des membranes cellulaires des couches vivantes de l'épiderme, sont peu à peu remplacés par un mélange composé en majeure partie d'acides gras, de cholestérol et de sphingolipides.

Ces lipides sont organisés en structures lamellaires spécifiques dont l'intégrité dépend non seulement de la qualité des fractions présentes mais aussi de leur proportion respective. Cette structure lamellaire des lipides du domaine lipidique de l'épiderme est responsable de la fluidité donc de la souplesse de la peau.

Les lipides sont également responsables des propriétés "barrière" de l'épiderme particulièrement du stratum corneum.

Les lipides épidermiques sont synthétisés principalement dans l'épiderme vivant. Ils sont principalement constitués de phospholipides, de sphingolipides, de cholestérol, d'acides gras libres, de triglycérides, d'esters du cholestérol et d'alcanes.

Les phospholipides sont essentiels pour la constitution des membranes cellulaires. Ils jouent un rôle important dans la médiation des signaux extracellulaires et la formation de chaînes aliphatiques libres utilisées pour la production d'énergie. Ils constituent un réservoir d'acides gras libres nécessaires à la constitution des sphingolipides.

Les sphingolipides (ou céramides) sont essentiels pour le maintien de la structure multilamellaire des lipides intercornéocytaires. Ils sont également essentiels pour les échanges en eau et la fonction "barrière" de l'épiderme.

Le cholestérol joue un rôle primordial dans l'hydratation cutanée et dans la fonction "barrière" de l'épiderme.

Les acides gras libres jouent un rôle majeur dans le maintien de la structure lamellaire des lipides du stratum corneum, ainsi que dans la constitution des membranes cellulaires où ils sont responsables de la fluidité membranaire mais également de processus physiologiques tels que le fonctionnement de récepteurs ou l'activité enzymatique.

On comprend alors le rôle essentiel que jouent les lipides de la peau et l'importance que revêt leur intégrité.

Or, dans certaines situations, qu'il s'agisse de pathologies spécifiques (dermite atopique), de vieillissement cutané, de vieillissement actinique, de peau sèche ou encore de fonction barrière altérée par des agressions répétées, physiques ou chimiques, l'épiderme humain présente des modifications de sa composition et/ou de sa synthèse lipidique.

Afin d'améliorer le contenu en lipide de l'épiderme et par conséquent d'agir sur la souplesse de la peau, deux voies d'action sont envisageables. La première est l'apport exogène de composés lipidiques par voie topique. La deuxième consiste à stimuler la synthèse des lipides endogènes. Il a ainsi été démontré qu'il était possible d'améliorer le profil lipidique des épidermes reconstruits en ajoutant de l'acide ascorbique (vitamine C) dans le milieu de culture (J. Invest. Dermatol. 109 :348-355, 1997). Toutefois, en raison de sa structure chimique (d'alpha-cétolactone), l'acide ascorbique est très sensible à certains paramètres de l'environnement comme la lumière, la chaleur et les milieux aqueux, en particulier les milieux alcalins et/ou aérobies. En raison de ces problèmes de stabilité, il est nécessaire d'utiliser de fortes concentrations d'acide ascorbique pour observer l'effet sur la peau d'une composition le contenant.

Par ailleurs, on connaît des dérivés de vitamine C qui sont plus stables que l'acide ascorbique lui-même. Ainsi, la demande EP-0 664 290 décrit des mono- et diesters d'acide cinnamique ou de l'un de ses dérivés avec l'acide ascorbique ou un dérivé. Ces composés peuvent être utilisés comme anti-oxydants dans des compositions cosmétiques ou pharmaceutiques, en vue notamment de protéger les lipides de la peau contre oxydation induite par les radicaux libres. D'autres dérivés d'acide ascorbique, qui sont des esters de (5,6-isopropylidène) ascorbyle, sont décrits dans les demandes JP-46 024 699 et JP-44 000 220. Des esters de l'acide palmitique et de l'acide ascorbique sont aussi décrits dans la demande EP-0 916 862, ainsi que des esters de l'acide linoléique et de l'acide ascorbique dans la demande WO-99128588. D'autres dérivés de l'acide pivaloïque et de l'acide ascorbique sont décrits dans la demande JP-61085308 pour leur effets blanchissants et hydratants. Enfin, d'autres dérivés d'acide ascorbique, tels que le le 2-O-α-D glucopyranosyl d'acide L-ascorbique (ou glucoside d'ascorbyle), sont disponibles dans le commerce. Le glucoside d'ascorbyle est notamment utilisé comme dépigmentant.

La demande EP-A-487404 décrit une composition dermatologique à action hydratante contenant le glucoside d'ascorbyle.
Le brevet US-A-5730972 décrit une composition cosmétique antiride et hydratante contenant un ester d'acide ascorbique et de saccharose ou le glucoside d'ascorbyle et un filtre UVA sulphonique hydrosoluble.
La demande EP-A-1077066 décrit une composition pour le traitement des maladies de peau comme l'atopie contenant des dérivés d'acide ascorbique à groupement phosphate ou glycoside.

La Demanderesse a maintenant découvert, de manière surprenante, que certains dérivés d'acide ascorbique, connus pour être plus stables que l'acide ascorbique lui-même, présentaient une efficacité supérieure à l'acide ascorbique dans la lipogénèse de l'épiderme, permettant ainsi leur utilisation à de plus faibles doses.

L'invention a donc pour objet l'utilisation cosmétique, pour améliorer la fonction barrière de la peau en vue d'améliorer l'hydratation de la peau, d'au moins un dérivé d'acide ascorbique choisi dans le groupe constitué par :
- le 2-hexadécanoate de (5,6-isopropylidène) ascorbyle
- le 2-cinnamate d'ascorbyle,
- le 2-férulate d'ascorbyle,
- le 2-férulate de (5,6-isopropylidène) ascorbyle,
- le 2-(4-acétoxyférulate) de (5,6-isopropylidène) ascorbyle,
ou un sel de ce dérivé,

Le 2-hexadécanoate de (5,6-isopropylidène) ascorbyle est particulièrement préféré.

Le composé 2-hexadécanoate de (5,6-isopropylidène) ascorbyle, ainsi que son procédé de préparation, sont décrits dans la demande JP-46 024 699. Les autres composés peuvent être préparés comme décrit dans la demande EP-0 664 290.

Les dérivés d'acide ascorbique précités sont utilisés dans, ou pour la fabrication, d'une composition comprenant généralement un milieu physiologiquement acceptable. Cette composition renferme avantageusement de 0,001 à 10% en poids, et de préférence de 0,01 à 1% en poids, de dérivé d'acide ascorbique.

La Demanderesse a démontré que ces composés augmentaient la synthèse des céramides épidermiques. Par "céramides épidermiques", on entend, selon la présente description, aussi bien les céramides de type I à VII, en particulier les céramides de type IV à VII, que les acylglucosylcéramides.

Ces propriétés des dérivés d'acide ascorbique selon l'invention sur la lipogénèse rendent la composition les contenant particulièrement bien adaptée à l'amélioration de la fonction barrière de la peau, qui permet elle-même une meilleure rétention d'eau dans la peau. Les dérivés d'acide ascorbique précités peuvent ainsi être utilisés dans une composition cosmétique hydratante, et la composition résultante dans un procédé cosmétique d'hydratation de la peau, comprenant l'application sur la peau de cette composition.

D'une façon plus générale, les dérivés d'acide ascorbique selon l'invention peuvent être utilisés à des fins cosmétiques, comme agents pour améliorer la souplesse de la peau et/ou l'aspect de surface de la peau (qui se manifeste notamment par des rugosités et la présence rides et ridules) et/ou lutter contre ou prévenir le vieillissement intrinsèque de la peau. Ils peuvent également être utiles pour protéger la peau contre les agressions, en particulier contre les effets des substances chimiques telles que les tensioactifs, des substances cosmétiques irritantes telles que les rétinoïdes, des agressions physiques telles que les frottements, et également contre les effets du froid ou du vent.

La composition selon l'invention peut aussi être utilisée pour fabriquer une préparation destinée à améliorer la fonction barrière de la peau. Une telle préparation peut être destinée au traitement de certaines pathologies impliquant une perturbation de la fonction barrière, telles que la dermite atopique ou séborrhéique.

Selon une autre forme d'exécution de l'invention, les dérivés d'acide ascorbique mentionnés précédemment peuvent être utilisés pour améliorer le contenu en lipides et/ou la fonction barrière des épidermes reconstruits. L'addition de l'un au moins de ces dérivés dans le milieu de culture des épidermes reconstruits permet ainsi de rapprocher ces épidermes de la structure de la peau humaine normale et, par la même, de rendre les tests *in vitro* (notamment les études de pénétration) réalisés sur ces épidermes plus prédictifs des phénomènes qui seront observés *in vivo.*

La composition de l'invention peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou, mieux, des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, en particulier sous forme de stick pour les lèvres lorsqu'elle est par exemple destinée à traiter les fissures labiales. Elle peut être utilisée comme produit de soin et/ou comme produit de maquillage pour la peau.

De façon connue, la composition de l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse, dans les vésicules lipidiques et/ou dans les nanoparticules. En tout état de cause, ces adjuvants, ainsi que leurs proportions, seront choisis de manière à ne pas nuire aux propriétés recherchées de l'association d'actifs selon l'invention.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-20, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, on peut utiliser notamment les agents kératolytiques et/ou desquamants, les agents dépigmentants, les filtres UV, les agents anti-radicaux libres et leurs mélanges. En cas d'incompatibilité, certains au moins des actifs peuvent être incorporés dans des sphérules, notamment des vésicules ioniques ou non-ioniques et/ou des nanoparticules (nanocapsules et/ou nanosphères), de manière à ce que les actifs incompatibles entre eux soient isolés les uns des autres dans la composition.

L'invention sera mieux comprise, et ses avantages ressortiront mieux, à la lumière des exemples suivants, qui sont donnés à titre illustratif, et sans limitation.

### Exemple 1 : Effet du glucoside d'ascorbyle sur la synthèse de lipides épidermiques

Le glucoside d'ascorbyle et l'acide ascorbique lui-même sont testés sur un équivalent de peau vendu par la société EPISKIN (LYON, France), après culture de celui-ci pendant 7 jours. Les milieux de culture et d'essais sont ceux inclus dans le kit vendu par le fournisseur. Chaque composé a été testé à 5 µg/ml et 0,05 µg/ml (en équivalent vitamine C) dans le milieu de culture. Les épidermes reconstruits sont traités durant cinq jours, le milieu de culture étant renouvelé toutes les 48 heures. Le témoin est constitué par un équivalent d'épiderme identique subissant une application topique de milieu de culture sans composé à tester.

A la fin de l'incubation, on détache l'équivalent d'épiderme de son support collagénique. La préparation des lipides de l'équivalent d'épiderme, et leur analyse par HPTLC ou chromatographie sur couche mince haute performance, sont réalisées selon la technique et avec les tampons décrits par M. Ponec (1991, Adv. Lipid Res. 24:83-117). En fin de migration, l'analyse densitométrique est réalisée à l'aide d'un lecteur densitométrique CAMAG TLC, modèle Scanner 3.

### Résultats et conclusion :

Les résultats sont présentés dans le Tableau 1 ci-dessous en pourcentage d'augmentation des céramides 4, 5, 6 et 7 par rapport au témoin non traité.

**TABLEAU 1**

| Variation de la teneur en Céramides 4 à 7 | | | |
|---|---|---|---|
| Concentration en équiv. vitamine C | | Peau traitée à l'acide ascorbique | Peau traitée au glucoside d'ascorbyle |
| 5 µg/ml | Céramides 4-7 | + 21 % | + 40% |
| | dont cér. 6-7 | - 17% | + 59% |
| 0,05 µg/ml | Céramides 4-7 | + 7% | +11% |
| | dont cér. 6-7 | + 15% | + 65% |

Ces résultats montrent une augmentation de la synthèse des lipides de l'épiderme (en particulier des céramides les plus polaires) qui est plus importante pour le glucoside d'ascorbyle que pour l'acide ascorbique testé à la même concentration.

### Exemple 2 :

Le test décrit dans l'Exemple 1 a été répété en utilisant différents dérivés d'acide ascorbique, appliqués durant 6 jours à 1,10 x 10⁻⁴M sur des épidermes reconstruits soumis à 7 jours de culture. La quantité de céramides épidermiques produite n'a toutefois pas été quantifiée. Au lieu de cela, une analyse densitométrique a été réalisée en utilisant un lecteur densitométrique CAMAG TLC^{®} Modèle Scanner 3.

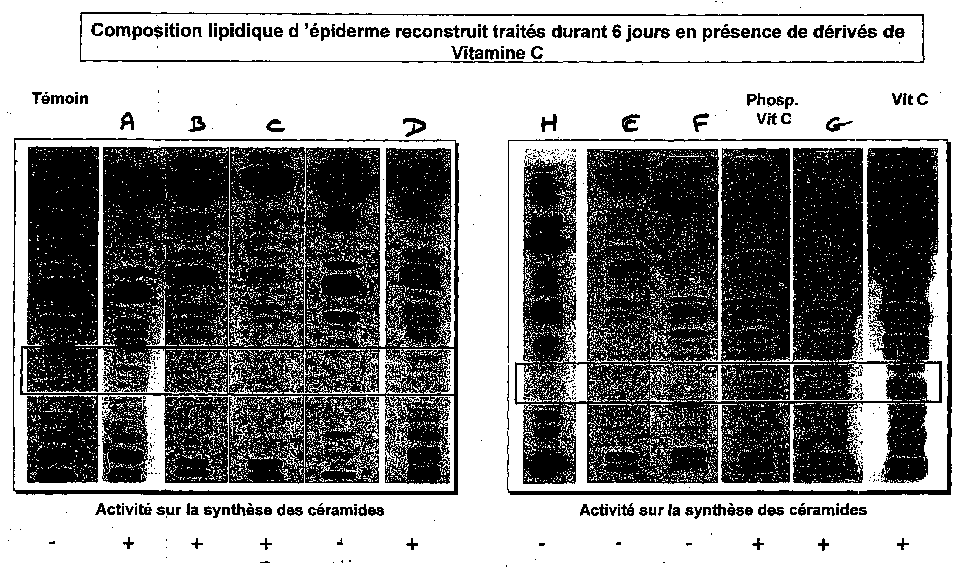

Le profil lipidique des épidermes traités est illustré sur la figure annexée sur laquelle :
- le composé A est le 2-O-α-D glucopyranosyl de l'acide (5,6-isopropylidène) ascorbique ;
- le composé B est le 2-cinnamate d'ascorbyle ;
- le composé C est le 2-férulate d'ascorbyle ;
- le composé D est le 2-hexadécanoate de (5,6-isopropylidène) ascorbyle ;
- le composé E est le 2,3-bis-(méthoxycarbonyl méthoxy)éther d'ascorbyle ;
- le composé F est le 2-ascorbyl sulfate de baryum ; et
- le composé G est le 2-benzoate de (5,6-isopropylidène) ascorbyle.

Le composé E est décrit, ainsi que son mode de préparation, dans la demande JP-07 206 840. Le composé F est disponible dans le commerce auprès de la société SIGMA.

La figure représente le contenu lipidique de l'échantillon d'épiderme reconstruit soit non traité (témoin), soit traité par l'acide ascorbique (vit C) ou par l'un des dérivés d'acide ascorbique A à G ci-dessus. La zone encadrée sur cette figure correspond aux céramides polaires (céramides IV à VII).

Il ressort de cette figure que l'application sur l'échantillon d'épiderme reconstruit des composés A, B, C, D et G augmente significativement la teneur de ces épidermes en céramides polaires, à la fois par rapport au témoin et par rapport à l'acide ascorbique, qui se traduit par un plus grand nombre de bandes et/ou par des bandes plus marquées. Au contraire, les composés E et F, qui sont également des dérivés d'acide ascorbique mais n'entrent pas dans la formule générale (I) des composés selon l'invention, n'ont pas d'effet significatif sur la lipogénèse de l'épiderme.

### Exemple 3 : composition cosmétique

On prépare une émulsion du type huile-dans-eau en procédant au mélange des ingrédients suivants :

| | |
|---|---|
| 2-cinnamate d'ascorbyle | 0,1 % |
| Polyéthylène glycol oxyéthyléné à 50 moles d'OE | 3 % |
| Diglycérol mono stéarate | 3 % |
| Huile de vaseline | 24 % |
| Alcool cétylique | 5 % |
| Eau q.s.p. | 100 % |

On obtient une crème qui peut être appliquée matin et/ou soir sur le visage pour améliorer la souplesse de la peau et lisser les rides et ridules.

### Exemple 4 : composition cosmétique

On prépare la composition suivante en procédant au mélange des ingrédients suivants :

| | |
|---|---|
| 2-férulate d'ascorbyle | 0,1 % |
| Huile de jojoba | 13 % |
| Mélange de méthyl et propyl paraben | 0,05 % |
| Sorbate de potassium | 0,3 % |
| Cyclopentadimethylsiloxane | 10 % |
| Alcool stéarylique | 1 % |
| Acide stéarique | 4 % |
| Stéarate de polyéthylène glycol | 3 % |
| Glycérol | 3 % |
| Eau q.s.p. | 100 % |

On obtient une crème qui est bien adaptée au traitement des peaux sèches.

Dans cet exemple, le 2-férulate d'ascorbyle peut être remplacé par une même quantité du 2-férulate de (5,6-isopropylidène) ascorbyle ou de 2-(4-actéoxyfërulate) de (5,6-isopropylidène) ascorbyle.

### Exemple 5 : composition cosmétique

On prépare une émulsion du type huile-dans-eau en procédant au mélange des ingrédients suivants :

| | |
|---|---|
| 2-hexadécanoate de (5,6-isopropylidène) ascorbyle | 0,1 % |
| Palmitate d'octyle | 10 % |
| Glycérol mono-isostéarate | 4 % |
| Huile de vaseline | 24 % |
| Vitamine E | 1 % |
| Glycérol | 3 % |
| Eau q.s.p. | 100 % |

On obtient une crème qui peut être appliquée matin et/ou soir sur le visage pour améliorer la souplesse de la peau et lisser les rides et ridules. Dans la composition ci-dessus, le 2-benzoate de (5,6-isopropylidène) ascorbyle peut être substitué au 2-hexadécanoate de (5,6-isopropylidène) ascorbyle.

## Revendications

1. Utilisation cosmétique, pour améliorer la fonction barrière de la peau en vue d'améliorer l'hydratation de la peau, d'au moins un dérivé d'acide ascorbique choisi dans le groupe constitué par :
- le 2-hexadécanoate de (5,6-isopropylidène) ascorbyle
- le 2-cinnamate d'ascorbyle,
- le 2-férulate d'ascorbyle,
- le 2-férulate de (5,6-isopropylidène) ascorbyle,
- le 2-(4-acétoxyférulate) de (5,6-isopropylidène) ascorbyle,
ou un sel de ce dérivé.

2. Utilisation selon la revendication 1, **caractérisée en ce que** le dérivé d'acide ascorbique est choisi parmi :
- le 2-hexadécanoate de (5,6-isopropylidène) ascorbyle
- le 2-férulate de (5,6-isopropylidène) ascorbyle,
- le 2-(4-acétoxyférulate) de (5,6-isopropylidène) ascorbyle,

3. Utilisation selon la revendication 1 ou 2, **caractérisée en ce que** le dérivé d'acide ascorbique est choisi parmi le 2-hexadécanoate de (5,6-isopropylidène) ascorbyle.

4. Utilisation selon l'une quelconque des revendications précédentes, **caractérisée en ce que** ladite composition comprend un milieu physiologiquement acceptable.

5. Utilisation selon l'une quelconque des revendications précédentes, **caractérisée en ce que** ladite composition renferme de 0,001 à 10% en poids, et de préférence de 0,01 à 1% en poids, de dérivé d'acide ascorbique.

## Claims

1. Cosmetic use, for improving the barrier function of the skin in order to improve moisturization of the skin, of at least one ascorbic acid derivative chosen from the group consisting of:
- (5,6-isopropylidene)ascorbyl 2-hexadecanoate,
- ascorbyl 2-cinnamate,
- ascorbyl 2-ferulate,
- (5,6-isopropylidene)ascorbyl 2-ferulate, and
- (5,6-isopropylidene)ascorbyl 2-(4-acetoxyferulate),
or a salt of this derivative.

2. Use according to Claim 1, **characterized in that** the ascorbic acid derivative is chosen from:
- (5,6-isopropylidene)ascorbyl 2-hexadecanoate,
- (5,6-isopropylidene)ascorbyl 2-ferulate,
- (5,6-isopropylidene)ascorbyl 2-(4-acetoxyferulate).

3. Use according to Claim 1 or 2, **characterized in that** the ascorbic acid derivative is chosen from (5,6-isopropylidene)ascorbyl 2-hexadecanoate.

4. Use according to any one of the preceding claims, **characterized in that** the said composition comprises a physiologically acceptable medium.

5. Use according to any one of the preceding claims, **characterized in that** the said composition contains from 0.001 to 10% by weight, and preferably from 0.01 to 1% by weight of the ascorbic acid derivative.

## Patentansprüche

1. Kosmetische Verwendung mindestens eines Ascorbinsäurederivats, das unter
- (5,6-Isopropyliden)ascorbyl-2-hexadecanoat
- Ascorbyl-2-cinnamat,
- Ascorbyl-2-ferulat,
- (5,6-Isopropyliden)ascorbyl-2-ferulat,
- (5,6-Isopropyliden)ascorbyl-2-(4-acetoxyfexulat),
ausgewählt ist, oder eines Salzes des Derivats, zur Verbesserung der Barrierefunktion der Haut mit dem Ziel, die Hydratisierung der Haut zu verbessern.

2. Verwendung nach Anspruch 1, **dadurch gekennzeichnet, dass** das Ascorbinsäurederivat ausgewählt ist unter
- (5,6-Isopropyliden)ascorbyl-2-hexadecanoat
- (5,6-Isopropyliden)ascorbyl-2-ferulat,
- (5,6-Isopropyliden)ascorbyl-2-(4-acetoxyferulat).

3. Verwendung nach einem der Ansprüche 1 oder 2, **dadurch gekennzeichnet, dass** das Ascorbinsäurederivat das (5,6-Isopropyliden)ascorbyl-2-hexadecanoat ist.

4. Verwendung nach einem der vorhergehenden Ansprüche, **dadurch gekennzeichnet, dass** die Zusammensetzung ein physiologisch akzeptables Medium enthält.

5. Verwendung nach einem der vorhergehenden Ansprüche, **dadurch gekennzeichnet, dass** die Zusammensetzung 0,001 bis 10 Gew.-% und besser 0,01 bis 1 Gew.-% des Ascorbinsäurederivats enthält.
